# EUROPEAN PATENT SPECIFICATION

(11) **EP 0 704 532 B1**
(45) Date of publication and mention of the grant of the patent: **11.09.2002**
(21) Application number: 95109019.0
(22) Date of filing: 12.06.1995
(51) Int. Cl.: C12N 15/62, C12N 15/12, C07K 14/51, C07K 14/78, C07K 14/47, C07K 14/495, A61K 38/18, A61K 38/39, C12N 1/21, A61K 47/48

(54) **Recombinant chimeric proteins and methods of use thereof**
Rekombinante Chimäre Proteine und Verfahren zur deren Verwendung
Protéines chimériques recombinantes et procédés pour leur utilisation

(30) Priority: 10.06.1994 US 259263
(43) Date of publication of application: 03.04.1996
(73) Proprietor: United States Surgical Corporation, Norwalk, Connecticut 06856 (US)
(72) Inventor: Gruskin, Elliott A., Killingworth, CT 06419 (US); Espino, Pearl, Madison, CT 06443 (US)
(74) Representative: Marsh, Roy David

(56) References cited:
- WO-A-90/03733
- WO-A-94/01483
- US-A- 5 302 701
- MOLECULAR ENDOCRINOLOGY, vol. 5, no. 1, pages 149-155, XP002000717 R.G.HAMMONDS ET AL.: "Bone-inducing activity of mature BMP-2b produced from a hybrid BMP-2a/2b precursor"

## Description

### BACKGROUND

### 1. Technical Field

Chimeric proteins and more particularly chimeric proteins having a domain which is derived from a physiologically active moiety and a domain derived from an extracellular matrix protein moiety are provided. Further provided are DNA constructs encoding such chimeric proteins and to methods for preparing such chimeric proteins using recombinant DNA technology. Methods for healing tissue including formation of bone and/or cartilage are also provided.

### 2. Description of Related Art

Chimeric proteins, also known as fusion proteins, are hybrid proteins which combine two or more precursor proteins or peptides through peptide bonds. Fusion proteins may be produced by recombinant technology, i.e., by fusing part of the coding sequence of one gene to the coding sequence of another gene. The fused gene may then be used to transform a suitable organism which then expresses the fusion protein. Such proteins are usually used to test the function of different domains of a protein molecule or to append a locater or binding peptide onto a protein or peptide of interest. For example, portions upstream and partially downstream of human, rat or mouse collagen genes have been fused to other proteins in an attempt to analyze characteristics of transcription. See, e.g., Rossouw, et al. DNA Sequences in the First Intron of the Human ProAlpha-1-I Collagen Gene Enhance Transcription, Journal of Biological Chemistry, 262 (31), pp. 15151-15157 (1987). Genomic imprinting effects have been analyzed by fusing the gene encoding human keratin 18 9 protein with the gene encoding beta-galactosidase (LacZ). See Thorex et al., Parent-Specific Expression of a Human Keratin 18/beta-galactosidase Fusion Gene in Transgenic Mice, Dev. Dyn. (United States), 195 (2) pp. 100-12 (Oct. 1992). European Patent Application 88302039 describes production and purification of a recombinant protein, e.g., collagen, a linker region which may encode a restriction site, and a binding protein for a substrate. The fusion protein is then contacted with a suitable substrate to which it binds and the protein may then be recovered, e.g., from a column.

Extracellular matrix proteins ("EMPs") are found in spaces around or near cells of multicellular organisms and are typically fibrous proteins of two functional types: mainly structural, e.g., collagen and elastin, and mainly adhesive, e.g., fibronectin and laminin. Collagens are a family of fibrous proteins typically secreted by connective tissue cells. Twenty distinct collagen chains have been identified which assemble to form a total of about ten different collagen molecules. A general discussion of collagen is provided by Alberts; et al., The Cell, Garland Publishing, pp. 802-823 (1989). Other fibrous or filamentous proteins include Type I IF proteins, e.g., keratins; Type II IF proteins, e.g., vimentin, desmin and glial fibrillary acidic protein; Type III IF proteins, e.g., neurofilament proteins; and Type IV IF proteins, e.g., nuclear laminins.

Physiologically active glycoproteins, proteins, peptides and proteoglycans are abundant in living things. Such glycoproteins, proteins, peptides and proteoglycans are involved in a diverse array of cellular or viral functions which include initiation or regulation of metabolism, catabolism, reproduction, growth and repair of various life forms. Physiologically active glycoproteins, proteins, peptides, and proteoglycans include therapeutically active glycoproteins, proteins, peptides, and proteoglycans such as hormones, growth factors, enzymes, ligands and receptors and fragments thereof. Therapeutically active substances include glycoproteins, proteins, peptides and proteoglycans which have been used in medicine and research, e.g., to achieve a beneficial result in relation to disease states, trauma and/or to increase efficiency of normal cellular functions. Examples of therapeutically active glycoproteins, proteins, peptides and proteoglycans include cellular regulatory factors such as interleukins, GCSF, erythropoietin, insulin, growth hormone, ACTH, thyroid hormones, various growth factors, osteogenic or osteoinductive factors, decorin and the like.

Osteogenic agents are any of a family of proteins or peptides that induce formation of bone and/or cartilage. Osteogenin, bone morphogenic protein ("BMP") or osteoinductive protein are other terms which describe proteins having bone inducing activity. BMPs are a family of related proteins that trigger the developmental cascade of bone differentiation by inducing mesenchymal stem cells to grow into a variety of tissues including bone, cartilage, and dentin. The activity of BMPs is particularly useful for repairing large bone defects which may not heal without clinical intervention.

Osteogenic agents have been isolated from demineralized mammalian bone tissue (see, e.g., U.S. Patent Nos. 4,294,753 and 4,761,471). Substantially pure BMPs have been produced by recombinant DNA techniques (see, e.g., U.S. Patent Nos. 5,106,748, 5,187,076, 5,141,905, 5,108,922, 5,166,058, and 5,116,738). U.S. Patent No. 5,168,050 describes the use of a DNA construct having a DNA sequence encoding the precursor portion of BMP-2A ligated to a DNA sequence encoding BMP-2B for obtaining improved expression of BMP-2B.

Certain methods have been employed for inducing formation of bone and/or cartilage with BMPs. When BMP is implanted in viable tissue without a delivery formulation, the BMP resorbs rapidly and does not effectively induce bone formation. Therefore, formulations for delivery or implantation of BMPs have been developed.

The following are examples of attempts to make delivery devices for BMPs. U.S. Patent No. 4,472,840 describes collagen and BMP conjugates or complexes in the form of microporous sponges to induce the formation of osseous tissue in animals or humans. U.S. Patent No. 4,975,527 describes enzyme-solubilized collagen as a carrier of bone morphogenic protein. U.S. Patent No. 4,563,489 describes delivery systems for BMP that are admixtures of biodegradable organic polymers such as polylactic acid and polyglycolic acid.

U.S. Patent No. 5,106,626 describes administration of osteogenic protein extracted from mammalian bone admixed with or absorbed on a matrix such as tricalcium phosphate, hydroxyapatite, thermoplastic polymer materials, collagen, plaster of paris, polylactic acid, polycaprolactic acid, or polyglycolic acid. U.S. Patent Nos. 5,011,691 and 5,250,302 describe methods of purifying osteogenic protein from mammalian bone and combining it with a matrix of porous material such as collagen, homopolymers or copolymers of glycolic acid and lactic acid, hydroxyapatite, or tricalcium phosphate.

It has been suggested that to prevent rapid resorption of BMP from a site of implantation, osteogenic sequestering agents may be used in connection with an admixture of osteogenic protein and a porous polymeric matrix. U.S. Patent No. 5,171,579 describes a composition of an admixture of an osteogenic protein, a porous particulate matrix and an osteogenic protein sequestering amount of blood clot. PCT WO 93/00050 describes an admixture of an osteogenic protein, a polymer matrix of poly (lactic acid), poly (glycolic acid), and copolymers of lactic acid and glycolic acid, and an osteogenic protein-sequestering material which may be alkylcellulose, hyaluronic acid, alginate, poly(ethylene glycol), polyoxyethylene oxide, carboxyvinyl polymer, poly(vinyl alcohol) or carboxymethylcellulose.

Notwithstanding the research done in the area of drug delivery devices, compositions which deliver a clinically effective dose of therapeutic agents over a predetermined period of time to precise target sites that combine easy handling for the medical practitioner with manufacturing convenience are still desirable. Elimination of the above-mentioned separate purified matrix materials, sequestering agents and substitution of more effective therapeutically active compositions would be advantageous.

### SUMMARY

Osteogenic chimeric proteins having a domain derived from at least one extracellular matrix protein and a domain derived from at least one bone morphogenic protein are provided. Suitable domains derived from cell regulatory factors include osteogenic domains.

Recombinant DNA constructs having DNA sequences encoding the above mentioned chimeric proteins are provided. Cloning vectors incorporating the above DNA constructs and cells transformed with the vectors are also provided. Therapeutic compositions incorporating the above-mentioned chimeric protein(s) and pharmaceutically acceptable vehicles are provided. For example, a drug delivery composition is provided which has a chimeric protein having a domain derived from a fibrous protein and a domain derived from a physiologically active glycoprotein, protein, peptide and/or proteoglycan.

Methods for preparing a DNA construct including a DNA sequence encoding an osteogenic agent operably linked to a DNA sequence encoding an extracellular matrix protein are provided. Also provided are methods of manufacturing osteogenic/extracellular matrix chimeric proteins by transforming a cell with a suitable cloning vector including a DNA construct encoding the osteogenic/extracellular matrix chimeric protein, culturing the cell in a suitable culture medium and isolating the chimeric protein from the culture medium.

In other embodiments, methods are provided for inducing formation of bone and involve contacting with a suitable locus an osteogenic chimeric protein. Suitable osteogenic chimeric proteins have a domain derived from one or more osteogenic agents and a domain derived from one or more extracellular matrix proteins. Further provided are methods for inducing bone formation by contacting the osteogenic chimeric protein with an implant at a suitable locus in viable tissue.

### BRIEF DESCRIPTION OF THE DRAWINGS

Fig. 1 depicts a nucleic acid sequence which encodes a BMP2B/collagen IA protein construct.
Fig. 2 depicts an amino acid sequence for a BMP2B/collagen IA chimeric protein.
Fig. 3 depicts a pMal cloning vector containing a polylinker cloning site.
Fig. 4 depicts a polylinker cloning site contained in a pMal cloning vector of Fig. 3.
Fig. 5 depicts a pMal cloning vector containing a BMP2B/collagen IA DNA construct.

### DETAILED DESCRIPTION

Osteogenic chimeric proteins according to the invention provide an integrated combination of a therapeutically active domain containing one or more therapeutically active moieties and an extracellular matrix protein domain containing one or more EMP moieties. The EMP domain provides an integral vehicle for delivery of the therapeutically active moiety to a target site. The two domains are linked covalently by one or more peptide bonds contained in a linker region. As used herein, integrated or integral means characteristics which result from the covalent association of one or more domains of the inventive chimeric proteins. The therapeutically active moieties disclosed herein are typically made of amino acids linked to form peptides, proteins, glycoproteins or proteoglycans. The inherent characteristics of EMPs are ideal for use as a vehicle for the therapeutic moiety. Examples of suitable EMPs are collagen, elastin, fibronectin, and fibrin. Fibrillar collagens (Type I, II and III) assemble into ordered polymers and often aggregate into larger bundles. Type IV collagen assembles into sheetlike meshworks. Elastin molecules form filaments and sheets in which the elastin molecules are highly cross-linked to one another and provides good elasticity and high tensile strength. The cross-linked, random-coiled structure of the fiber network allows it to stretch and recoil like a rubber band. Fibronectin is a large fibril forming glycoprotein, which, in one of its forms, consists of highly insoluble fibrils cross-linked to each other by disulfide bonds. Fibrin is an insoluble protein formed from fibrinogen by the proteolytic activity of thrombin during the normal clotting of blood.

The molecular and macromolecular morphology of the above EMPs defines networks or matrices to provide substratum or scaffolding in integral covalent association with the therapeutically active moiety. The networks or matrices formed by the EMP domain provide an environment particularly well suited for ingrowth of autologous cells involved in growth, repair and replacement of existing tissue. The integral therapeutically active moieties covalently bound within the networks or matrices provide maximum exposure of the active agents to their targets to elicit a desired response.

Implants formed of or from the present chimeric proteins provide sustained release activity in or at a desired locus or target site. Unlike the above-described compositions discussed in the Background which incorporate a vehicle not covalently linked to an EMP, the therapeutically active domain of the present chimeric protein is not free to separately diffuse or otherwise be transported away from the vehicle which carries it, absent cleavage of peptide bonds. Consequently, chimeric proteins provide an effective anchor for therapeutic activity which allows the activity to be confined a target location for a prolonged duration. Because the supply of therapeutically active agent does not have to be replenished as often, smaller amounts of therapeutically active agent may be used over the course of therapy. Consequently, certain advantages provided by the inventive chimeric proteins are a decrease or elimination of local and systemic side effects, less potentiation or reduction in therapeutic activity with chronic use, and minimization of drug accumulation in body tissues with chronic dosing.

Use of recombinant technology allows manufacturing of nonimmunogenic chimeric proteins. The DNA encoding both the therapeutically active moiety and EMP moiety should preferably be derived from the same species as the patient being treated to avoid an immunogenic reaction. For example, if the patient is human, the therapeutically active moiety as well as the EMP moiety is preferably derived from human DNA.

Osteogenic/EMP chimeric proteins provide biodegradable and biocompatible agents for inducing bone formation at a desired site. In one embodiment a BMP moiety is covalently linked with an EMP to form a chimeric protein. The BMP moiety induces osteogenesis and the extracellular matrix protein moiety provides an integral substratum or scaffolding for the BMP moiety and cells which are involved in reconstruction and growth. Compositions containing the BMP/EMP chimeric protein provide effective sustained release delivery of the BMP moiety to desired target sites. The method of manufacturing such an osteogenic agent is efficient because the need for extra time consuming steps such as purifying EMP and then admixing it with the purified BMP are eliminated. An added advantage of the BMP/EMP chimeric protein results from the stability created by the covalent bond between BMP and the EMP, i.e., the BMP portion is not free to separately diffuse away from the EMP, thus providing a more stable therapeutic agent.

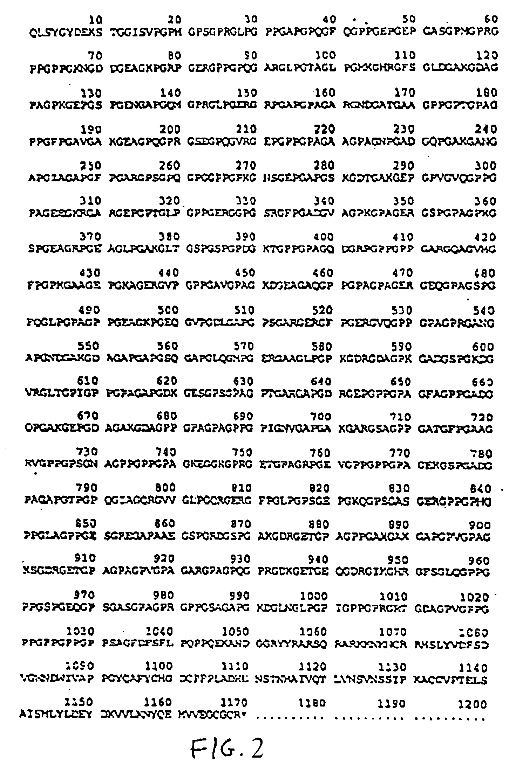

Bone morphogenic proteins are class identified as BMP-1 through BMP-9. A preferred osteogenic protein for use in human patients is human BMP-2B. A BMP-2B/collagen IA chimeric protein is illustrated in Fig.2. The protein sequence illustrated in Fig.2 includes a collagen helical domain depicted at amino acids 1-1057 and a mature form of BMP2B at amino acids 1060-1169. The physical properties of the chimeric protein are dominated in part by the EMP component. In the case of a collagen moiety, a concentrated solution of chimeric protein will have a gelatinous consistency that allows easy handling by the medical practitioner. The EMP moiety acts as a sequestering agent to prevent rapid desorption of the BMP moiety from the desired site and provide sustained release of BMP activity. As a results the BMP moiety remains at the desired site for a period of time necessary to effectively induce bone formation. The EMP moiety also provides a matrix which allows a patient's autologous cells, e.g., chondrocytes and the like, which are normally involved in osteogenesis to collect therein and form an autologous network for new tissue growth. The gelatinous consistency of the chimeric protein also provides a useful and convenient therapeutic manner for immobilizing active BMP on a suitable vehicle or implant for delivering the BMP moiety to a site where bone growth is desired.

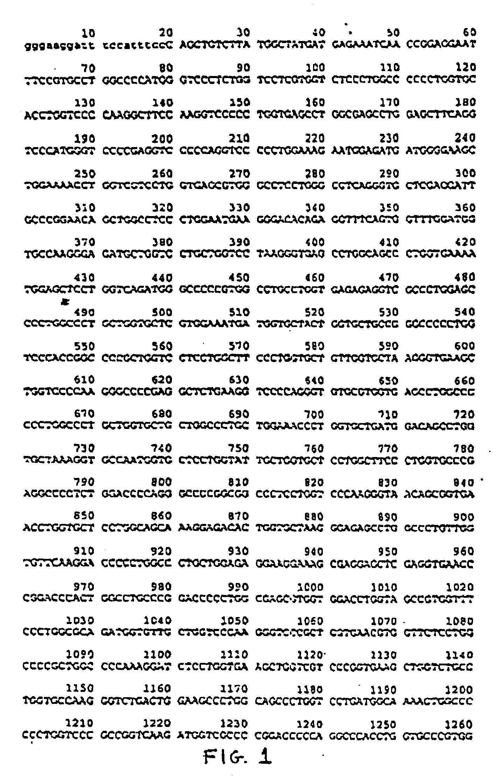

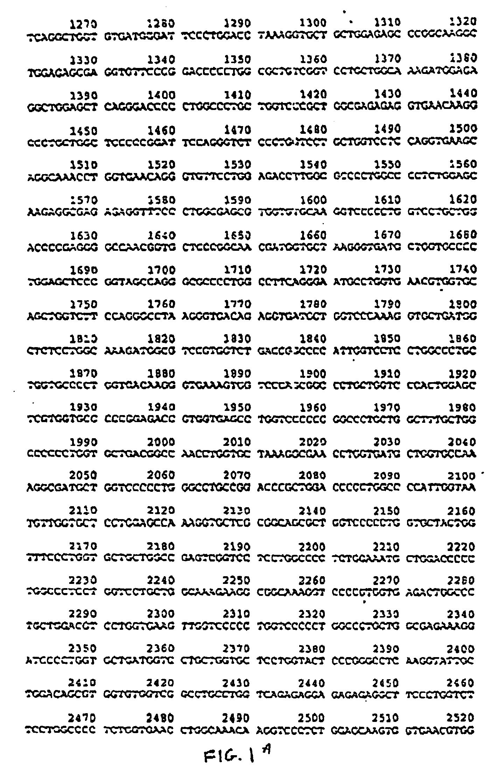

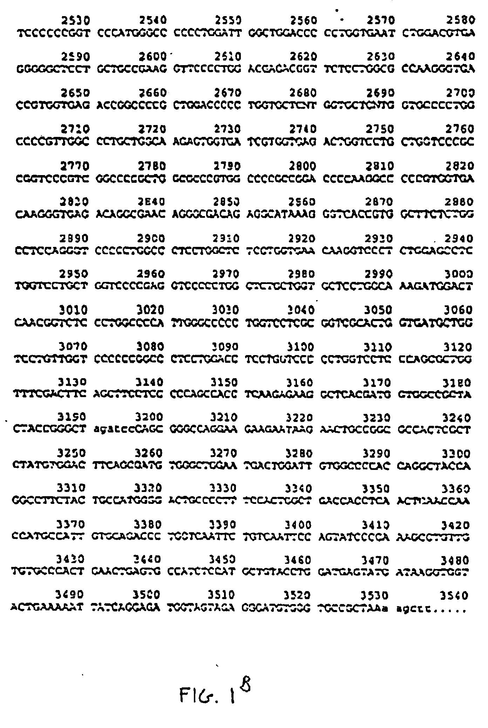

The BMP moiety and the EMP moiety are optionally linked together by linker sequences of amino acids. Examples of linker sequences used are illustrated within the sequences depicted in Figs. 1-2 and described in more detail below. Linker sequences may be chosen based on particular properties which they impart to the chimeric protein. For example, amino acid sequences such as Ile-Glu-Gly-Arg and Leu-Val-Pro-Arg are cleaved by Factor Xa and Thrombin enzymes, respectively. Incorporating sequences which are cleaved by proteolytic enzymes into chimeric proteins provides cleavage at the linker site upon exposure to the appropriate enzyme and separation of the two domains into separate entities. It is contemplated that numerous linker sequences can be incorporated into any of the chimeric proteins.

In another embodiment, a chimeric DNA construct includes a gene encoding an osteogenic protein or a fragment thereof linked to a gene encoding an EMP or a fragment thereof. The gene sequences for various BMPs are known, see, e.g., U.S. Patent Nos. 4,294,753, 4,761,471, 5,106,748, 5, 187, 076, 5,141,905, 5,108,922, 5,166,058, 5,116,738 and 5,168,050. A BMP-2B gene for use with this invention is synthesized by ligating oligonucleotides encoding a BMP protein. The oligonucleotides encoding BMP-2B are synthesized using an automated DNA synthesizer (Beckmen Oligo-1000). In a preferred embodiment, the nucleotide sequence encoding the BMP is maximized for expression in E. coli. This is accomplished by using E. coli utilization tables to translate the sequence of amino acids of the BMP into codons that are utilized most often by E. coli. Alternatively, native DNA encoding BMP isolated from mammals including humans may be purified and used.

The BMP gene and the DNA sequence encoding an extracellular matrix protein are cloned by standard genetic engineering methods as described in Maniatis et al., Molecular Cloning: A Laboratory Manual, Cold Spring Harbor 1982.

The DNA sequence corresponding to the helical region of collagen I(a) is cloned from a human fibroblast cell line. Two sets of polymerase chain reactions are carried out using cDNA prepared by standard methods from AG02261A cells. The first pair of PCR primers include a 5' primer bearing an XmnI linker sequence and a 3 primer bearing the BsmI site at nucleotide number 1722. The resulting PCR product consists of sequence from position 1 to 1722. The second pair of primers includes the BsmI site at 1722 and a linker sequence at the 3 end bearing a BglII site. The resulting PCR products consists of sequence from position 1722 to 3196. The complete helical sequence is assembled by standard cloning techniques. The two PCR products are ligated together at the BsmI site, and the combined clone is inserted into any vector with XmnIBglII sites of XmnI-BamHI sites such as pMALc2-vector.

To clone the BMP-2B gene, total cellular RNA is isolated from human osteosarcoma cells (U-20S) by the method described by Robert E. Farrel Jr. (Academic Press, CA, 1993 pp. 68-69). The integrity of the RNA is verified by spectrophotometric analysis and electrophoresis through agarose gels. Typical yields of total RNA are 50 µg from a 100mm confluent tissue culture dish. The RNA is used to generate cDNA by reverse transcription using the Superscript pre-amplification system by Gibco BRL. The cDNA is used as template for PCR amplification using upstream and downstream primers specific for BMP-2B (GenBank HUMBMP2B accession # M22490). The resulting PCR product consists of BMP-2B sequence Eom position 1289-1619. The PCR product is resolved by electrophoresis through agarose gels, purified with gene clean (BIO 101) and ligated into pMal-c2 vector (New England Biolabs). The helical domain of human collagen I(a) chain is cloned in a similar manner. However, the total cellular RNA is isolated from a human fibroblast cell line (AG02261A human skin fibroblasts).

A chimeric BMP/EMP DNA construct is obtained by ligating a synthetic BMP gene to a DNA sequence encoding an EMP such as collagen, fibrin, fibronectin, elastin or laminin. However, the invention is not limited to these particular proteins. Fig. 1 illustrates a DNA construct which encodes a BMP-2B/collagen IA chimeric protein. The coding sequence for an EMP may be ligated upstream and/or downstream and in-frame with a coding sequence for the BMP. The DNA encoding an EMP may be a portion of the gene or an entire EMP gene. Furthermore, two different EMPs may be ligated upstream and downstream from the BMP.

The BMP-2B/collagen IA chimeric protein illustrated in Fig. 1 includes an XmnI linker sequence at base pairs (bp) 1-19, a collagen helical domain (bp 20-3190), a BglII/BamHI linker sequence (bp 3191-3196), a mature form of BMP-2B (bp 3197-3529) and a HindIII linker sequence (bp 3530-3535).

Any combination of bone morphogenic protein and matrix protein sequences are contemplated including repeating units, or multiple arrays of each segment in any order. Incorporation of fragments of both matrix and bone morphogenic proteins is also contemplated. For example, in the case of collagen, only the helical domain may be included. Other matrix proteins have defined domains, such as laminin, which has EGF-like domains. In these cases, specific functionalities can be chosen to achieve desired effects. Moreover, it may be useful to combine domains from disparate matrix proteins, such as the helical region of collagen and the cell attachment regions of fibronectin. In the case of growth factors, specific segments have been shown to be removed from the mature protein by post translational processing. Chimeric proteins can be designed to include only the mature biologically active region. For example, in the case of BMP-2B only the final 110 amino acids are found in the active protein.

The coding sequence for an EMP may be ligated upstream and/or downstream and in-frame with a coding sequence for decorin. The DNA encoding the EMP may encode a portion or fragment of the EMP or may encode the entire EMP. Likewise, the DNA encoding decorin may be a fragment thereof or the entire gene. Furthermore, two or more different EMPs may be ligated upstream from the DNA encoding decorin moiety.

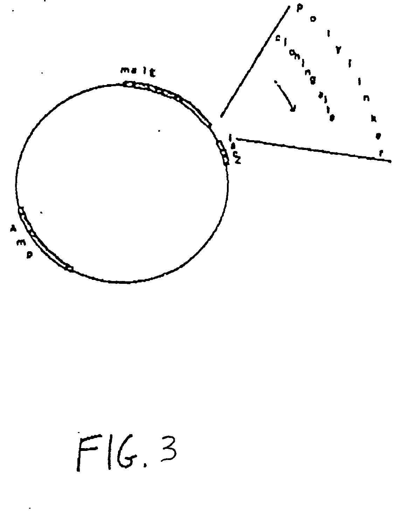

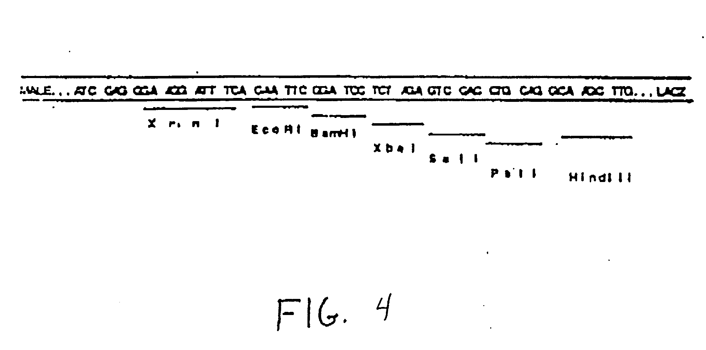

Any of the above described chimeric DNA constructs may be incorporated into a suitable cloning vector. Fig. 3 depicts a pMal cloning vector containing a polylinker cloning site. Preferred cloning vectors are the plasmids pMal-p2 and pMal-c2 (commercially available from New England Biolabs). The desired chimeric DNA construct is incorporated into a polylinker sequence of the plasmid which contains certain useful restriction endonuclease sites which are depicted in Fig. 4. The pMal-p2 polylinker sequence has XmnI, EcoRI, BamHI, HindIII, XbaI, SalI and PstI restriction endonuclease sites which are depicted in Fig. 4. The polylinker sequence is digested with an appropriate restriction endonuclease and the chimeric construct is incorporated into the cloning vector by ligating it to the DNA sequences of the plasmid. The chimeric DNA construct may be joined to the plasmid by digesting the ends of the DNA construct and the plasmid with the same restriction endonuclease to generate "sticky ends" having 5' phosphate and 3' hydroxyl groups which allow the DNA construct to anneal to the cloning vector. Gaps between the inserted DNA construct and the plasmid are then sealed with DNA ligase. Other techniques for incorporating the DNA construct into plasmid DNA include blunt end ligation, poly(dA.dT)tailing techniques, and the use of chemically synthesized linkers. An alternative method for introducing the chimeric DNA construct into a cloning vector is to incorporate the DNA encoding the extracellular matrix protein into a cloning vector already containing a gene encoding a therapeutically active moiety.

The cloning sites in the above-identified polylinker site allow the cDNA for the collagen IA/BMP-2B chimeric protein illustrated in Fig. 1 to be inserted between the XmnI and the HindIII sites.

Plasmids containing the chimeric DNA construct are identified by standard techniques such as gel electrophoresis. Procedures and materials for preparation of recombinant vectors, transformation of host cells with the vectors, and host cell expression of polypeptides are described in Maniatis et al., Molecular Cloning: A Laboratory Manual, Cold Spring Harbor 1982. Generally, prokaryotic or eukaryotic host cells may be transforrned with the recombinant DNA plasmids. Transformed host cells may be located through phenotypic selection genes of the cloning vector which provide resistance to a particular antibiotic when the host cells are grown in a culture medium containing that antibiotic.

Transformed host cells are isolated and cultured to promote expression of the chimeric protein. The chimeric protein may then be isolated from the culture medium and purified by various methods such as dialysis, density gradient centrifugation, liquid column chromatography, isoelectric precipitation, solvent fractionation, and electrophoresis. However, purification of the chimeric protein by affinity chromatography is preferred whereby the chimeric protein is purified by ligating it to a binding protein and contacting it with a ligand or substrate to which the binding protein has a specific affinity.

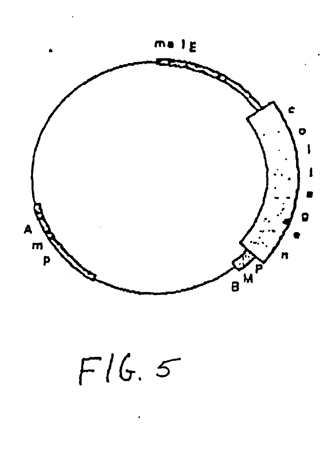

In order to obtain more effective expression of mammalian or human eukaryotic genes in bacteria (prokaryotes), the mammalian or human gene should be placed under the control of a bacterial promoter. A protein fusion and purification system is employed to obtain the chimeric protein. Preferably, any of the above-described chimeric DNA constructs is cloned into a pMal vector at a site in the vector's polylinker sequence. As a result, the chimeric DNA construct is operably fused with the malE gene of the pMal vector. The malE gene encodes maltose binding protein (MBP). Fig. 5 depicts a pMal cloning vector containing a BMP/collagen DNA construct. A spacer sequence coding for 10 asparagine residues is located between the malE sequence and the polylinker sequence. This spacer sequence insulates MBP from the protein of interest. The pMal vector containing any of the chimeric DNA constructs fused to the malE gene is transformed into E. coli. This technique utilizes the PtaC promoter of the malE gene.

The E. coli is cultured in a medium which induces the bacteria to produce the maltose binding protein fused to the chimeric protein. The MBP contains a 26 amino acid N-terminal signal sequence which directs the MBP-chimeric protein through the E. coli cytoplasmic membrane. The protein can then be purified from the periplasm. Alternatively, the pMal-c2 cloning vector can be used with this protein fusion and purification system. The pMal-c2 vector contains an exact deletion of the malE signal sequence which results in cytoplasmic expression of the fusion protein. A crude cell extract containing the fusion protein is prepared and poured over a column of amylose resin. Since MBP has an affinity for the amylose it binds to the resin. Alternatively, the column can include any substrate for which MBP has a specific affinity. Unwanted proteins present in the crude extract are washed through the column. The MBP fused to the chimeric protein is eluted from the column with a neutral buffer containing maltose or other dilute solution of a desorbing agent for displacing the hybrid polypeptide. The purified MBPchimeric protein is cleaved with a protease such as factor Xa protease to cleave the MBP from the chimeric protein. The pMal-p2 plasmid has a sequence encoding the recognition site for protease factor Xa which cleaves after the amino acid sequence Isoleucine-Glutamic acid-Glycine-Arginine of the polylinker sequence.

The chimeric protein is then separated from the cleaved MBP by passing the mixture over an amylose column. An alternative method for separating the MBP from the chimeric protein is by ion exchange chromatography. This system yields up to 100mg of MBP-chimeric protein per liter of culture. See Riggs, P., in Ausebel, F.M., Kingston, R.E., Moore, D.D., Seidman, J.G., Smith, J.A., Struhl, K. (eds.) Current Protocols in Molecular Biology, Supplement 19 (16.6.116.6.10) (1990) Green Associates/Wiley Interscience, New York, New England Biolabs (cat # 800-65S 9pMALc2) pMal protein fusion and purification system (See also European Patent No. 286 239 which discloses a similar method for production and purification of a protein such as collagen.)

Other protein fusion and purification systems may be employed to produce chimeric proteins. Prokaryotes such as E. coli are the preferred host cells for expression of the chimeric protein. However, systems which utilize eukaryote host cell lines are also acceptable such as yeast, human, mouse, rat, hamster, monkey, amphibian, insect, algae, and plant cell lines. For example, HeLa (human epithelial), 3T3 (mouse fibroblast), CHO (Chinese hamster ovary), and SP 2 (mouse plasma cell) are acceptable cell lines. The particular host cells that are chosen should be compatible with the particular cloning vector that is chosen.

Another acceptable protein expression system is the Baculovirus Expression System manufactured by Invitrogen of San Diego, California. Baculoviruses form prominent crystal occlusions within the nuclei of cells they infect. Each crystal occlusion consists of numerous virus particles enveloped in a protein called polyhedrin. In the baculovirus expression system, the native gene encoding polyhedrin is substituted with a DNA construct encoding a protein or peptide having a desired activity. The virus then produces large amounts of protein encoded by the foreign DNA construct. The preferred cloning vector for use with this system is pBlueBac III (obtained from Invitrogen of San Diego, California). The baculovirus system utilizes the *Autograph californica* multiple nuclear polyhidrosis virus (AcMNPV) regulated polyhedrin promoter to drive expression of foreign genes. AcMNPV is isolated from a moth called the California looper. The chimeric gene, i.e., the DNA construct encoding the chimeric protein, is inserted into the pBlueBac III vector immediately downstream from the baculovirus polyhedrin promoter.

The pBlueBac III transfer vector contains a B-galactosidase reporter gene which allows for identification of recombinant virus. The B-galactosidase gene is driven by the baculovirus ETL promoter (PETL) which is positioned in opposite orientation to the polyhedrin promoter (PpH) and the multiple cloning site of the vector. Therefore, recombinant virus coexpresses B-galactosidase and the chimeric gene.

*Spodoptera frugeperda (Sf9)* insect cells are then cotransfected with wild type viral DNA and the pBlueBac III vector containing the chimeric gene. Recombination sequences in the pBlueBac III vector direct the vector's integration into the genome of the wild type baculovirus. Homologous recombination occurs resulting in replacement of the native polyhednn gene of the baculovirus with the DNA constuct encoding the chimeric protein. Wild type baculovirus which do not contain foreign DNA express the polyhedrin protein in the nuclei of the infected insect cells. However, the recombinants do not produce polyhedrin protein and do not produce viral occlusions. Instead, the recombinants produce the chimeric protein.

Alternative insect host cells for use with this expression system are Sf21 cell line derived from *Spodoptera frugeperda* and High Five cell lines derived from *Trichoplusia ni.*

Other acceptable cloning vectors include phages, cosmids or artificial chromosomes. For example, bacteriophage lambda is a useful cloning vector. This phage can accept pieces of foreign DNA up to about 20,000 base pairs in length. The lambda phage genome is a linear double stranded DNA molecule with single stranded complementary (cohesive) ends which can hybridize with each other when inside an infected host cell. The lambda DNA is cut with a restriction endonuclease and the foreign DNA, e.g. the DNA to be cloned, is ligated to the phage DNA fragments. The resulting recombinant molecule is then packaged into infective phage particles. Host cells are infected with the phage particles containing the recombinant DNA. The phage DNA replicates in the host cell to produce many copies of the desired DNA sequence.

Cosmids are hybrid plasmid/bacteriophage vectors which can be used to clone DNA fragments of about 40,000 base pairs. Cosmids are plasmids which have one or more DNA sequences called "cos" sites derived from bacteriophage lambda for packaging lambda DNA into infective phage particles. Two cosmids are ligated to the DNA to be cloned. The resulting molecule is packaged into infective lambda phage particles and transfected into bacteria host cells. When the cosmids are inside the host cell they behave like plasmids and multiply under the control of a plasmid origin of replication. The origin of replication is a sequence of DNA which allows a plasmid to multiply within a host cell.

Yeast artificial chromosome vectors are similar to plasmids but allow for the incorporation of much larger DNA sequences of about 400,000 base pairs. The yeast artificial chromosomes contain sequences for replication in yeast. The yeast artificial chromosome containing the DNA to be cloned is transformed into yeast cells where it replicates thereby producing many copies of the desired DNA sequence. Where phage, cosmids, or yeast artificial chromosomes are employed as cloning vectors, expression of the chimeric protein may be obtained by culturing host cells that have been transfected or transformed with the cloning vector in a suitable culture medium.

Chimeric proteins disclosed herein are intended for use in treating mammals or other animals. The therapeutically active moieties described above, namely, osteogenic agents such as BMPs, and/or fragments thereof are all to be considered as being or having been derived from cellular regulatory factors. The chimeric proteins and DNA constructs which incorporate a domain derived from one or more cellular regulatory factors can be used for in vivo therapeutic treatment, in vitro research or for diagnostic purposes in general.

When used in vivo, formulations containing the inventive chimeric proteins may be placed in direct contact with viable tissue, including bone, to induce or enhance growth, repair and/or replacement of such tissue. This may be accomplished by applying a chimeric protein directly to a target site during surgery. It is contemplated that minimally invasive techniques such as endoscopy are to be used to apply a chimeric protein to a desired location. Formulations containing the chimeric proteins disclosed herein may consist solely of one or more chimeric proteins or may also incorporate one or more pharmaceutically acceptable adjuvants.

In an alternate embodiment, any of the above-described chimeric proteins may be contacted with, adhered to, or otherwise incorporated into an implant such as a drug delivery device or a prosthetic device. Chimeric proteins may be microencapsulated or macroencapsulated by liposomes or other membrane forming materials such as alginic acid derivatives prior to implantation and then implanted in the form of a pouchlike implant. The chimeric protein may be microencapsulated in structures in the form of spheres, aggregates of core material embedded in a continuum of wall material or capillary designs. Microencapsulation techniques are well known in the art and are described in the Encyclopedia of Polymer Science and Engineering, Vol. 9, pp. 724 et seq. (1980). Chimeric proteins may also be coated on or incorporated into medically useful materials such as meshes, pads, felts, dressings or prosthetic devices such as rods pins, bone plates, artificial joints, artificial limbs or bone augmentation implants. The implants may, in part, be made of biocompatable materials such as glass, metal, ceramic, calcium phosphate or calcium carbonate based materials. Implants having biocompatible biomaterials are well known in the art and are all suitable for use. Implant biomaterials derived from natural sources such as protein fibers, polysaccharides, and treated naturally derived tissues are described in the Encyclopedia of Polymer Science and Engineering, Vol. 2, pp. 267 et seq. (1989). Synthetic biocompatible polymers are well known in the art and are also suitable implant materials. Examples of suitable synthetic polymers include urethanes, olefins, terephthalates, acrylates, polyesters and the like. Other acceptable implant materials are biodegradable hydrogels or aggregations of closely packed particles such as polymethylmethacrylate beads with a polymerized hydroxyethyl methacrylate coating. See the Encyclopedia of Polymer Science and Engineering, Vol. 2, pp. 267 et seq. (1989).

The chimeric protein provides a useful way for immobilizing or coating a cellular regulatory factor on a pharmaceutically acceptable vehicle to deliver the cellular regulatory factor to desired sites in viable tissue. Suitable vehicles include those made of bioabsorbable polymers, biocompatible nonabsorbable polymers, lactoner putty and plaster of Paris. Examples of suitable bioabsorbable and biocompatible polymers include homopolymers, copolymers and blends of hydroxyacids such as lactide and glycolide, other absorbable polymers which may be used alone or in combination with hydroxyacids include dioxanones, carbonates such as trimethylene carbonate, lactones such as caprolactone, polyoxyaLkylenes, and oxylates. See the Encyclopedia of Polymer Science and Engineering, Vol. 2, pp. 230 et seq. (1989).

These vehicles may be in the form of beads, particles, putty, coatings or film vehicles. Diffusional systems in which a core of chimeric protein is surrounded by a porous membrane layer are other acceptable vehicles.

The following examples should be considered as illustrative of certain embodiments disclosed herein but should not be considered as limiting the inventive disclosure.

### EXAMPLE I

### Cloning BMP-2B/collagen IA DNA segment constructs

Obtaining PCR products for BMP-2B and Collagen I(a): The chimeric gene encoding the BMP-2B/Collagen I(a) fusion protein is assembled from PCR products. The PCR primers are designed to provide restriction sites on the 5' and 3' ends that facilitate later ligation steps. The 5' and 3' ends of the BMP-2B PCR product contain BamHI and HindIII restriction sites respectively. The 5' and 3' ends of the Collagen I(a) PCR product contain XmnI and BglII restriction sites respectively. Amplification is carried out on template cDNA synthesized from total cellular RNA using standard methods. PCR reactions for BMP-2B and Collagen I(a) use cDNA prepared from U-20S and AG02261A cell lines respectively. After amplification and purification, the PCR products are ligated into PCR II vectors. Positive clones are identified by screening plasmids for the correct molecular weight. The clones are verified by DNA sequencing using standard methods. The BMP-2B PCR product is excised from PCRII by restriction digestion with BamHI and HindIII and the Collagen I(a) segment was excised from PCRII using XmnI and BglII. The restriction digest reactions are resolved by electrophoresis through agarose gels and the DNA fragments with the BMP-2B and Collagen I(a) sequences are purified with gene clean (BIO 101).

### EXAMPLE II

### Construction of cloning vector incorporating DNA constructs of Example 1

Ligation of BMP-2B and Collagen I(a) segments into the pMal-c2 expression vector: The pMal-c2 vector is treated with BamHI and Hind3, resolved by electrophoresis through an agarose gel and purified by standard methods. The BMP-2B segment with matching BamHI and Hind3 restriction sites on the 5' and 3' ends is ligated into pMal-c2 and transformants are screened for the insert by standard techniques. Positive clones are verified by DNA sequencing and designated pMal-c2 BMP. To complete the construction, pMal-c2-BMP is digested with XmnI and BamHI and the Collagen I(a) segment which is digested with XmnI and BglII is ligated into those sites by standard methods (BamHI and BglII produce compatible termini). Positive clones are verified by DNA sequencing and designated pMal-CB. See Fig. 5.

### EXAMPLE III

### Transformation of E. Coli and Expression of Chimeric Genes in E. coli

Expression plasmid pMal-CB (Collagen-BMP2B Chimera), are used to transform E.coli HB 101 using standard techniques. To express protein, a 50 ml culture of E.coli harboring one of the expression vectors is inoculated into 1L of LB broth and incubated with agitation at 37°C. When the A₆₀₀ is 0.5±0.1, 0.1M IPTG is added to a final concentration of 1.5-15 mM. The culture is maintained at 37°C until the A₆₀₀ is 1.3 to 1.8 and the E.coli is harvested by centrifugation at 4000xg. The cell pellets are resuspended in 7.5 ml 20 mM Tris HCl pH 7.5, 200 mM NaCl, 1 mM EDTA (hereinafter "column buffer") and frozen in a dry ice/ethanol bath. The frozen cell pellets are thawed at 4°C, then sonicated on ice until the cells are disrupted. Cell debris is removed by centrifugation at 9,000xg at 4°C for 30 minutes. The supernatant fraction contains the E.coli crude cell lysate which is analyzed for protein production by SDS-PAGE. The recombinant protein products produced from the pMal vector is a-fusion protein with MBP (maltose binding protein). The MBP segment is included to allow a single step purification of the protein.

The crude lysate is passed over an amylose column containing ml of resin/3 mg of recombinant protein (expected yield). The column is washed with 8 volumes of column buffer and the column flow through is reapplied to the column. Another 8 volumes of column buffer is used to wash the column. The fusion protein is eluted from the column using column buffer containing 10 mM Maltose. Fractions containing the recombinant chimeric protein are identified by the BCA protein assay (Pierce) and verified by SDS-PAGE. The fractions that contain the protein are pooled

The MBP segment of the purified protein is cleaved from the collagen-growth factor chimera by treatment with factor Xa (New England Biolabs) at room temperature for 24 hours. The collagen-growth factor chimera is separated from the MBP segment by chromatography through an amylose column. The column flow through contains the collagen-growth factor chimera, which is analyzed by SDS-PAGE. Typical yield of purified protein range from 10-50 mg/liter of E.coli culture.

### EXAMPLE III

### Expression of a Collagen-Growth Factor Chimeric Genes in Sf9 Cells

A useful alternative to the E.coli expression system is Baculovirus. The gene for the collagen-bone morphogenic protein chimeras is modified to include an ATG start codon at the 5' end and a TAA stop codon at the 3' end. The transcriptional unit is ligated into the baculoviral transfer vector pBlueBac III Invitrogen). The resulting transfer vector is verified by DNA sequencing. The collagen-bone morphogenic protein chimera gene is transferred into the baculovirus genome (AcMNPV) by the standard in vivo recombination method. The pBlueBacIII transfer vector containing the collagen-bone morphogenic protein chimera gene is cotransfected into Sf9 cells by standard methods. Recombinant viral plaques that are blue are selected and isolated by several rounds of reinfection. Pure recombinant baculovirus is verified by DNA sequencing. The recombinant virus containing the collagen-bone morphogenic protein chimera gene is used to infect suspension cultures of Sf9 cells and optimal protein expression is determined at 48-72 hours post-infection. The protein product is recovered from the culture medium and analyzed by SDS-PAGE.

The claims which follow identify embodiments of the invention additional to those described in detail above.

## Claims

1. A chimeric DNA construct comprising a domain derived from a DNA sequence encoding a bone morphogenic protein or fragment thereof and a domain derived from a DNA sequence encoding an extracellular matrix protein, wherein the protein encoded by said chimeric DNA construct has osteogenic activity.

2. A chimeric DNA construct according to claim 1, wherein said extracellular matrix protein is selected from the group consisting of collagen, laminin, fibronectin, elastin and fibrin.

3. The DNA construct according to claim 1 or 2, wherein said BMP protein comprises BMP-2B.

4. A cloning vector comprising a DNA construct according to any one of claims 1 to 3.

5. A cloning vector according to claim 4, wherein said cloning vector is selected from the group consisting of plasmids, phages, cosmids and artificial chromosomes.

6. A cloning vector according to claim 4 or 5, wherein said cloning vector is pMal p2, or pMal-c2.

7. A cell transformed by a cloning vector according to any one of claims 4 to 6.

8. A cell according to claim 7 wherein said cell is selected from the group consisting of E. Coli, HeLa, 3T3, CHO, SP2, Sf9, Sf21, and High Five.

9. An osteogenic chimeric protein comprising a domain derived from a bone morphogenic protein or fragment thereof and a domain derived from an extracellular matrix protein.

10. A chimeric protein according to claim 9, wherein said extracellular matrix protein is selected from the group consisting of collagen, fibronectin, elastin, laminin and fibrin.

11. A method of manufacturing a chimeric bone morphogenic protein /extracellular matrix protein comprising: transforming a cell with the vector according-to any one of claims 4 to 6; culturing said cell in a suitable culture medium; and obtaining said chimeric bone morphogenic protein/extracellular matrix protein from said culture medium.

12. A pharmaceutical composition comprising an osteogenic chimeric protein comprising a domain derived from a bone morphogenic protein or fragment thereof and a domain derived from an extracellular matrix protein and a pharmaceutically acceptable vehicle.

13. A pharmaceutical composition according to claim 12, wherein said extracellular matrix protein is selected from the group consisting of collagen, fibronectin, elastin and fibrin.

14. A pharmaceutical composition according to claim 12 or 13, wherein said vehicle comprises a material selected from the group consisting of bioabsorbable polymers, bicompatible nonabsorbable polymers, lactoner putty and plaster of Paris.

15. A pharmaceutical composition according to claim 14, wherein said material is selected from the group consisting of lactide, glycolide, trimethylene carbonate, dioxanone, caprolactone, polymethylmethacrylate and hydroxyethylmethacrylate.

16. A method of preparing a DNA construct according to claim 1 comprising: providing DNA which encodes a bone morphogenic protein or fragment thereof;
providing DNA which encodes an extracellular matrix protein or fragment thereof; and operably linking said bone morphogenic protein or fragment thereof encoding DNA to said extracellular matrix protein or fragment thereof encoding DNA to form a chimeric DNA construct.

17. Use of a chimeric protein according to any one of claim 9 or 10 or a pharmaceutical composition according to any one of claims 12 to 15 for the manufacture of a medicament for the prevention or treatment of disease.

18. Use of a chimeric protein according to claim 9 or 10 or a pharmaceutical composition according to any one of claims 12 to 15 for the manufacture of a osteogenic agent.

19. Use of a chimeric protein according to claim 9 or 10 or a pharmaceutical composition according to any one of claims 12 to 15, for the manufacture of a medicament for inducing bone and/or cartilage formation.

## Patentansprüche

1. Chimäres DNA-Konstrukt, das eine Domäne umfaßt, die von einer DNA-Sequenz stammt, die ein Knochenmorphogeneseprotein oder ein Fragment davon kodiert, und eine Domäne, die von einer DNA-Sequenz stammt, die ein extrazelluläres Matrixprotein kodiert, wobei das durch das chimäre DNA-Konstrukt kodierte Protein osteogene Wirkung hat.

2. Chimäres DNA-Konstrukt gemäß Anspruch 1, wobei das extrazelluläre Matrixprotein aus der Gruppe, die aus Kollagen, Laminin, Fibronectin, Elastin und Fibrin besteht, ausgewählt ist.

3. DNA-Konstrukt gemäß Anspruch 1 oder 2, wobei das BMP-Protein BMP-2B umfaßt.

4. Klonierungsvektor, der ein DNA-Konstrukt gemäß einem der Ansprüche 1 bis 3 umfaßt.

5. Klonierungsvektor gemäß Anspruch 4, wobei der Klonierungsvektor aus der Gruppe, die aus Plasmiden, Phagen, Cosmiden und artifiziellen Chromosomen besteht, ausgewählt ist.

6. Klonierungsvektor gemäß Anspruch 4 oder 5, wobei der Klonierungsvektor pMal-p2 oder pMal-c2 ist.

7. Mit einem Klonierungsvektor gemäß einem der Ansprüche 4 bis 6 transformierte Zelle.

8. Zelle gemäß Anspruch 7, wobei die Zelle aus der aus E. coli, HeLa, 3T3, CHO, SP2, Sf9, Sf21 und High Five bestehenden Gruppe ausgewählt ist.

9. Osteogenes chimäres Protein, das eine Domäne, die von einem Knochenmorphogeneseprotein oder einem Fragment davon stammt, und eine Domäne, die von einem extrazellulären Matrixprotein stammt, umfaßt.

10. Chimäres Protein gemäß Anspruch 9, wobei das extrazelluläre Matrixprotein aus der Gruppe, die aus Kollagen, Fibronectin, Elastin, Laminin und Fibrin besteht, ausgewählt ist.

11. Verfahren zur Herstellung eines chimären Knochenmorphogeneseproteins/extrazellulären Matrixproteins, das umfaßt: Transformieren einer Zelle mit dem Vektor gemäß einem der Ansprüche 4 bis 6; Kultivieren der Zelle in einem geeigneten Kulturmedium; und Gewinnen des chimären Knochenmorphogeneseproteins/extrazellulären Matrixproteins aus dem Kulturmedium.

12. Pharmazeutische Zusammensetzung, die ein osteogenes chimäres Protein umfaßt, das eine Domäne, die von einem Knochenmorphogeneseprotein oder einem Fragment davon stammt, und eine Domäne, die von einem extrazellulären Matrixprotein stammt sowie ein pharmazeutisch akzeptables Vehikel umfaßt.

13. Pharmazeutische Zusammensetzung gemäß Anspruch 12, wobei das extrazelluläre Matrixprotein aus der Gruppe, die aus Kollagen, Fibronectin, Elastin und Fibrin besteht, ausgewählt ist.

14. Pharmazeutische Zusammensetzung gemäß Anspruch 12 oder 13, wobei das Vehikel ein Material, das aus der Gruppe, die aus bioabsorbierbaren Polymeren, biokompatiblen nicht-absorbierbaren Polymeren, Lactoner Kitt (lactoner putty) und Pariser Gips besteht, umfaßt.

15. Pharmazeutische Zusammensetzung gemäß Anspruch 14, wobei das Material aus der Gruppe, die aus Lactid, Glycolid, Trimethylencarbonat, Dioxanon, Caprolacton, Polymethylmethacrylat und Hydroxyethylmethacrylat besteht, ausgewählt ist.

16. Verfahren zur Herstellung eines DNA-Konstrukts gemäß Anspruch 1, umfassend:
Zurverfügungstellen einer DNA, die ein Knochenmorphogeneseprotein oder ein Fragment davon kodiert;
Zurverfügungstellen einer DNA, die ein extrazelluläres Matrixprotein oder ein Fragment davon kodiert; und
funktionsfähiges Verbinden der DNA, die das Knochenmorphogeneseprotein oder Fragment davon kodiert, mit der DNA, die das extrazelluläre Matrixprotein oder Fragment davon kodiert, um ein chimäres DNA-Konstrukt zu bilden.

17. Verwendung eines chimären Proteins gemäß einem der Ansprüche 9 oder 10 oder einer pharmazeutischen Zusammensetzung gemäß einem der Ansprüche 12 bis 15 zur Herstellung eines Medikaments zur Krankheitsprävention oder -behandlung.

18. Verwendung eines chimären Proteins gemäß Anspruch 9 oder 10 oder einer pharmazeutischen Zusammensetzung gemäß einem der Ansprüche 12 bis 15 zur Herstellung eines osteogenen Mittels.

19. Verwendung eines chimären Proteins gemäß Anspruch 9 oder 10 oder einer pharmazeutischen Zusammensetzung gemäß einem der Ansprüche 12 bis 15 zur Herstellung eines Medikaments zum Induzieren der Knochen- und/oder Knorpelgewebebildung.

## Revendications

1. Construction d'ADN chimérique comprenant un domaine dérivé d'une séquence d'ADN codant pour une protéine morphogénique osseuse ou un fragment de celle-ci et un domaine dérivé d'une séquence d'ADN codant pour une protéine de matrice extracellulaire, où la protéine encodée par ladite construction d'ADN chimérique a une activité ostéogénique.

2. Construction d'ADN chimérique selon la revendication 1, où ladite protéine de matrice extracellulaire est sélectionnée dans le groupe consistant en collagène, laminine, fibronectine, élastine et fibrine.

3. Construction d'ADN selon la revendication 1 ou 2, où ladite protéine BMP comprend BMP-2B.

4. Vecteur de clonage comprenant une construction d'ADN selon l'une quelconque des revendications 1 à 3.

5. Vecteur de clonage selon la revendication 4 où ledit vecteur de clonage est sélectionné dans le groupe consistant en plasmides, phages, cosmides et chromosomes artificiels.

6. Vecteur de clonage selon la revendication 4 ou 5, où ledit vecteur de clonage est pMal-p2, ou pMalc2.

7. cellule transformée par un vecteur de clonage selon l'une quelconque des revendications 4 à 6.

8. Cellule selon la revendication 7 où ladite cellule est sélectionnée dans le groupe consistant en E.coli, HeLa, 3T3, CHO, SP2, Sf9, Sf21, et High Five.

9. Protéine chimérique ostéogénique comprenant un domaine dérivé d'une protéine morphogénique de l'os ou un fragment de celle-ci et un domaine dérivé d'une protéine de matrice extracellulaire.

10. Protéine chimérique selon la revendication 9, où ladite protéine de matrice extracellulaire est sélectionnée dans le groupe consistant en collagène, fibronectine, élastine, laminine et fibrine.

11. Méthode de fabrication d'une protéine morphogénique de l'os/protéine de matrice extracellulaire chimérique comprenant: la transformation d'une cellule avec le vecteur selon l'une quelconque des revendications 4 à 6; la culture de ladite cellule dans un milieu approprié de culture; et l'obtention de ladite protéine morphogénique osseuse/protéine de matrice extracellulaire chimérique dudit milieu de culture.

12. Composition pharmaceutique comprenant une protéine chimérique ostéogénique comprenant un domaine dérivé d'une protéine morphogénique osseuse ou son fragment et un domaine dérivé d'une protéine de matrice extracellulaire et un véhicule pharmaceutiquement acceptable.

13. Composition pharmaceutique selon la revendication 12, où ladite protéine de matrice extracellulaire est sélectionnée dans le groupe consistant en collagène, fibronectine, élastine et fibrine.

14. Composition pharmaceutique selon la revendication 12 ou 13, où ledit véhicule comprend une matière sélectionnée dans le groupe consistant en polymères bioabsorbables, polymères non absorbables biocompatibles, mastic de lactone et plâtre de Paris.

15. Composition pharmaceutique selon la revendication 14, où ladite matière est sélectionnée dans le groupe consistant en lactide, glycolide, carbonate de triméthylène, dioxanone, caprolactone, polyméthylméthacrylate et hydroxyéthylméthacrylate.

16. Méthode de préparation d'une construction d'ADN selon la revendication 1 consistant à prévoir un ADN qui code pour une protéine morphogénique osseuse ou un fragment de celle-ci;
à prévoir un ADN qui code pour une protéine de matrice extracellulaire ou un fragment de celle-ci; et à enchaîner opérativement ladite protéine morphogénique osseuse ou son fragment codant pour un ADN à ladite protéine de matrice extracellulaire ou son fragment codant pour un ADN pour former une construction d'ADN chimérique.

17. Utilisation d'une protéine chimérique selon l'une quelconque des revendications 9 ou 10 ou d'une composition pharmaceutique selon l'une quelconque des revendications 12 à 15 pour la fabrication d'un médicament pour la prévention ou le traitement d'une maladie.

18. Utilisation d'une protéine chimérique selon la revendication 9 ou 10 ou d'une composition pharmaceutique selon l'une quelconque des revendications 12 à 15 pour la fabrication d'un agent ostéogénique.

19. Utilisation d'une protéine chimérique selon la revendication 9 ou 10 ou d'une composition pharmaceutique selon l'une quelconque des revendications 12 à 15 pour la fabrication d'un médicament pour induire la formation de l'os et/ou du cartilage.
